(19) Europäisches Patentamt
European Patent Office
Office européen des brevets

(11) Veröffentlichungsnummer: **0 133 971**
**B1**

(12) # EUROPÄISCHE PATENTSCHRIFT

(45) Veröffentlichungstag der Patentschrift: **06.04.88**

(21) Anmeldenummer: **84108945.1**

(22) Anmeldetag: **27.07.84**

(51) Int. Cl.[4]: **C 07 D 493/10**, C 07 H 7/06, A 61 K 31/70, A 61 K 31/335, C 12 P 17/18, C 12 P 19/44 // (C07D493/10, 311:00, 307:00)

(54) **Antibiotikum, dessen Herstellung sowie dessen Verwendung.**

(30) Priorität: **28.07.83 US 518233**

(43) Veröffentlichungstag der Anmeldung: **13.03.85 Patentblatt 85/11**

(45) Bekanntmachung des Hinweises auf die Patenterteilung: **06.04.88 Patentblatt 88/14**

(84) Benannte Vertragsstaaten: **AT BE CH DE FR GB IT LI LU NL SE**

(56) Entgegenhaltungen:
**EP-A-0 024 189**
**EP-A-0 048 585**
**CH-A-637 963**
**GB-A-2 113 671**
**US-A-4 269 971**

**Adv. Appl. Microbiol. 22, 177-223 (1977)**

(73) Patentinhaber: **F. HOFFMANN- LA ROCHE & CO. Aktiengesellschaft, CH- 4002 Basel (CH)**

(72) Erfinder: **Liu, Chao- Min, 36 Rockledge Place, Cedar Grove, N.J. (US)**
Erfinder: **Westley, John, 91 South Mountain Avenue, Cedar Grove, N.J. (US)**

(74) Vertreter: **Freiherr von Pechmann, Eckehart, Patentanwälte Wuesthoff- v. Pechmann- Behrens- Goetz Schweigerstrasse 2, D-8000 München 90 (DE)**

EP 0 133 971 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Polyätherionophorantibiotikum der Formel

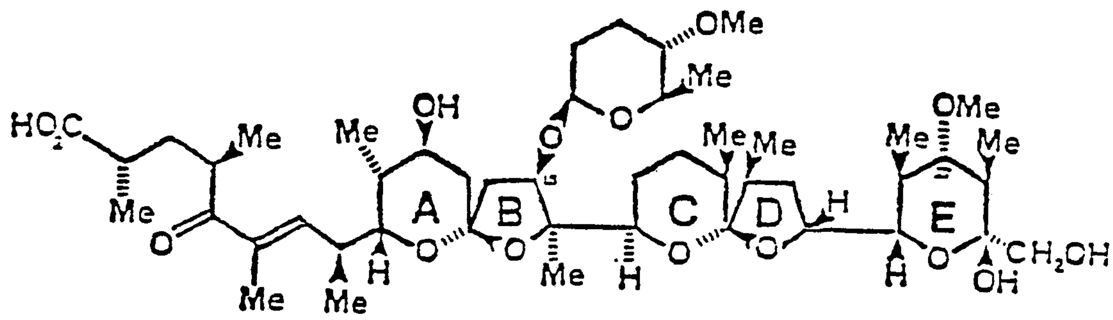

I

worin Me Methyl bedeutet, sowie dessen Salze.

Die Verbindung der Formel I sowie deren Salze zeigen Aktivität als antibakterielle und als antikokzidiostatische Mittel.

Antibiotikum X-14934A ist die Bezeichnung eines kristallinen Antibiotikums, welches durch einen Streptomycesorganismus gebildet wird; lyophilisierte Proben dieses Organismus wurden beim U.S. Department of Agriculture, Agriculture Research Service, Northern Regional Research Laboratories (NRRL), Peoria, Illinois hinterlegt. Die Kultur hat von NRRL die Identifikationsnummer NRRL 15518 erhalten.

Antibiotikum X-14934A ist ein Polyätherantibiotikum und bildet eine vielzahl von Salzen. Diese Salze werden aus der freien Säure des Antibiotikums nach bestens bekannten Methoden für Polyätherantibiotika gebildet; z. B. durch Waschen der freien Säure in Lösung mit einer geeigneten Base oder einem Salz. Beispiele von basischen Substanzen welche fähig sind, Salze zu bilden, umfassen Alkalimetallbasen, wie Natriumhydroxid, Kaliumhydroxid, Lithiumhydroxid und dergleichen; Erdalkalimetallbasen, wie Calciumhydroxid, Bariumhydroxid und dergleichen; und Ammoniumhydroxid. Alkali- oder Erdalkalimetallsalze, welche zur Salzbildung fähig sind, können Anionen wie Carbonate, Bicarbonate und Sulfate umfassen.

Beispiele von organischen Basen, welche mit dem Polyätherantibiotikum Salze bilden können, umfassen niedrige primäre, sekundäre und tertiäre Alkylamine, und Hydroxyalkylamine, wie Äthylamin, Isopropylamin, Diäthylamin, Methyl-n-butylamin, Äthanolamin und Diäthanolamin.

Morphologische Charakteristiken

Ein repräsentativer Stamm von Streptomyces X-14934A hat die folgenden Charakteristiken:

1. Mikroskopische Charakteristiken. Die Kultur X-14934 wächst in Agarmedien verschiedener Zusammensetzung und liefert ein submerges Mycel, welches in den Agar eintritt und mit dem Alter nicht fragmentiert, sowie ein Luftmycel, welches teilweise in Sporenketten differenziert. Diese Ketten sind spiralförmig und haben mehr als je 10 Sporen. Die Sporen können einzeln nicht gesehen werden, da die gesamte Kette umhüllt ist und nach der Hydrierung gefaltet wird. Die Sporen sind glatt und messen im Durchschnitt 0,6 µm bis 1,1 µm.

Papierchromatographische Analyse des gesamten Zellhydrolysats zeigt die Anwesenheit von L,L-Diaminopimelinsäure, welches die Identifikation dieses Organismus als Stamm des Genus Streptomyces bestätigt.

2. Macroskopische charakteristiken. In der nachfolgenden Tabelle sind die Charakteristiken des Wachstums, des Ausmasses der Sporulation der Farbe der Luftmasse und des reversen Mycels in verschiedenen Medien angegeben. Die Daten wurden nach 14-tägiger Inkubation bei 28°C aufgezeichnet.

| Medium | Ausmass des Wachstums Sporulationsgrad | Farbe der Luftmasse (1) | Farbe des reversen Mycels (1) |
|---|---|---|---|
| Hefe-Malzextrakt (ISP-2) | reichliches Wachstum; keine Sporenbildung | Muschelweiss (b) | Strohfarben (2 fb) |
| Hafermehlagar (ISP-3) | reichliches Wachstum; hygroskopisch; gute Sporulation | beigebraun (3 ig) mit weissen Flecken | silbergrau (3 fe) mit weissen Flecken |
| Anorganische Salze-Stärkeagar (ISP-4) | reichliches Wachstum und gute Sporenbildung | silbergrau (3 fe) | pastellgelb (1 1/2 fb) |
| Glycerin-Asparaginagar (ISP-5) | schwaches Wachstum; spärliche Sporenbildung | weiss (a) | weiss (a) |

(1) Der verwendete Farbcod ist derjenige des Color Harmony Manual vierte Ausgabe, Container Corporation von America, 1958.

3. Physiologische Charakteristiken. Der Stamm X-14934 verwendet Glukose, L-Arabinose, Sucrose, i-Inositol, Mannit, Fruktose, Rhamnose und Raffinose und weniger effektiv, Xylose als einzige Kohlenstoffquellen für das Wachstum. Die Verwendung von Cellulose ist negativ.

Die Bildung von $H_2S$, angezeigt durch Dunkelwerden in Peptonhefeextrakteisenagar (ISP 6), ist positiv, und eine schwarze Farbe (Melanin) entwickelt sich in Tyrose enthaltendem Medium (ISP 7). Nitratreduktiom ist negativ. Gelatin, Stärke und Caseinhydrolyse sind positiv. Bei einer Natriumchloridkonzentration von 3.5 % oder höher ist kein Wachstum zu beobachten.

4. Vergleich mit bekannten Streptomyces Spezies. Auf Basis der Farbe der Sporenmasse, der Form der Sporenkette, der Sporenoberfläche und der Bildung vom Melanoidpigmenten, sowie des hygroskopischen Charakters der Kolonien auf gewissen Medien sowie des Kohlenstoffverbrauchstests kann Stamm X-14934 der Spezies Streptomyces hygroscopicus zugeordnet werden.

Der Streptomyces X-14934 wie er hierin beschrieben wird, umfasst alle Stämme von Streptomyces, welche eine Verbindung, wie sie in der vorliegenden Anmeldung beansprucht werden, bildet und welcher nicht endgültig von diesem NRRL-Stamm seinen Subkulturen einschliesslich Mutanten und Varianten unterschieden werden kann. Die beanspruchte Verbindung wird hierin beschrieben und - nachdem deren Identifikation bekannt ist - ist es einfach, Stämme, die diese Verbindung herstellen, von andern Stämmen zu unterscheiden.

Wenn Streptomyces X-14934 unter geeigneten Bedingungen wächst, produziert er das Antibiotikum X-14934A. Eine Fermentationsbrühe enthaltend Streptomyces X-14934 wird hergestellt durch Inokulieren von Sporenmycel des Organismus, welcher das Antibiotikum herstellt, in ein geeignetes Medium und dann durch Kultivation unter aeroben Bedingungen. Für die Herstellung des Antibiotikums ist eine Kultivation auf einem Festmedium möglich, doch ist für die Herstellung grösserer Mengen die Kultivation in einem flüssigen Medium bevorzugt. Die Temperatur der Kultivation kann über einen weiten Bereich variieren, der Organismus kann wachsen bei einer Temperatur won 20-35°, doch ist eine Temperatur von 26-30°C und ein praktisch neutraler pH bevorzugt. Bei der submergen aeroben Fermentation des Organismus zur Bildung des Antibiotikums X-14934A kann das Medium als Kohlenstoffquelle ein käuflich erhältliches Glyceridöl oder einen Kohlenwasserstoff, wie Glycerin, Glukose, Maltose, Lactose, Dextrin, Stärke etc. in reiner oder roher Form, und als Quelle für Stickstoff ein organisches Material wie Sojabohnenmehl, Schlempe, Erdnussmehl, Baumwollmehl, Fleischextrakt, Pepton, Fischmehl, Hefeextrakt, Kornlaugenflüssigkeit etc. und falls erwünscht anorganische Quellen für Stickstoff, wie Nitrate und Ammoniumsalze, und Mineralsalze, wie Ammoniumsulfat, Magnesiumsulfat, und dergleichen enthalten. Das Medium kann weiterhin Natriumchlorid, Kaliumchlorid, Kaliumphosphat und dergleichen und Puffersubstanzen, wie Natriumcitrat, Kalziumcarbonat oder Phosphat, sowie Spuren von Schwermetallen enthalten. In belüfteten submergen Kulturverfahren wird ein Antischaummittel, wie flüssiges Paraffin, Fettsäuren oder Silikonverbindungen, verwendet. Bei der Herstellung des Antibiotikums X-14934A kann mehr als eine Art von Kohlenstoff-Stickstoffquelle oder Antischaummittel verwendet werden.

Das Antibiotikum X-14934A hat eine Toxizität ($LD_{50}$) bei Mäusen von 25 mg/kg (p.o.) und 7,75 mg/kg (i.p.) als Monohydratnatriumsalz.

Aus den Dokumenten EP-A-0 048 585, EP-A-0 024 189, CH-A-637 963, GB-A-2 113 671, US-A-4 269 971 sowie Adv. Appl. Microbiol. 22, 177-223 (1977) sind verschiedene polyzyklische Polyätherantibiotika bekannt, wobei das in der US-A-4 269 971 beschriebene Polyätherantibiotikum TM-531 dem hier beschriebenen Antibiotikum X-14934A strukturell am nächsten kommt. Das hier beschriebene Antibiotikum X-14934A unterscheidet sich vom vorbekannten Polyätherantibiotikum TM-531 im wesentlichen dadurch, dass der Tetrahydrapyranylrest sich beim Antibiotikum X-14934A am Tetrahydrofuranylring B befindet, während er sich beim vorbekannten Polyätherantibiotikum TM-531 am Tetrahydrofuranylring C befindet. Weiterhin zeigt das neue Antibiotikum X-14934A am Tetrapyranylring E ein anderes Substitutionsmuster als das Antibiotikum TM-531.

Im weiteren hat sich gezeigt, dass das neue Polyätherantibiotikum X-14934A im Gegensatz zum vorbekannten Polyätherantibiotikum TM-531 auch gegen Hefen, insbesondere gegen Candida albicans wirksam ist.

Die folgenden Beispiele zeigen eine Methode zur Herstellung des Antibiotikums X-14934A.

**Beispiel 1**

Streptomyces X-14934 wird an einer Stärkecaseinagarschrägkultur der folgenden Zusammensetzung (g/l destilliertes Wasser) wachsen gelassen.

| | |
|---|---|
| Lösliche Stärke | 10,0 |
| Casein | 1,0 |
| $K_2HPO_4$ | 0,5 |
| $MgSO_4$ | 0,5 |
| Agar | 20,0 |

der pH wird mit Natriumhydroxid vor dem Autoklavieren auf 7,4 eingestellt.

Eine Schrägkultur wird mit einer Kultur von X-14934 beimpft und während 7-14 Tagen bei 28°C inkubiert. Ein Klumpen Agar enthaltend Sporen und Mycel der inkubierten Schrägkultur wird zur Herstellung eines vegetativen Inoculums durch Impfen in einem 500 ml Erlenmeyer-Kolben enthaltend 100 ml Medium der folgenden Zusammensetzung (g/l Leitungswasser) verwendet.

| | |
|---|---|
| Soyalose | 10,0 |
| Cerelose | 20,0 |
| $Na_2SO_4$ | 1,0 |
| $CaCO_3$ | 0,2 |
| $CoCl_2.6H_2O$ | 0,001 |

der pH wird vor der Sterilisation auf 6.0 eingestellt.

Das beimpfte Medium wird während 4 Tagen auf einem Rotationsschüttler, der bei 250 UPM arbeitet, bei 28°C inkubiert. Zwei 30 ml-Portionen der erhaltenen Kultur werden zur Beimpfung von zwei 6 l Erlenmeyer-Kolben verwendet, welche je 2 l des oben erwähnten Mediums enthalten. Diese beimpften 6 l Erlenmeyer-Kolben werden während 4 Tagen bei 28°C an einem Rotationsschüttler, der bei 250 UPM arbeitet, inkubiert. Die erhaltenen 4 Liter vegetatives Wachstum werden zur Beimpfung eines 100-Gallonenfermentors enthaltend 60 Gallonen Produktionsmedium der folgenden Zusammensetzung (g/l Leitungswasser) verwendet.

| | | |
|---|---|---|
| Cerelose | 20,0 | |
| Soyalose | 10,0 | der pH wird vor der Sterili- |
| $Na_2SO_4$ | 1,0 | sation auf 6,0 eingestellt |
| $CaCO_3$ | 0,2 | |
| $CoCl_2.6H_2O$ | 0,001 | |

SAG 4130 Antischaummittel (werden sofern benötigt während der Fermentation zugegeben).

Der beimpfte Tank wird mit 3 Kubik Fuss/min. belüftet und mit einem Rührer mit 280 UPM gerührt. Die Fermentation wird während 97 Tagen bei 28°C durchgeführt.

**Beispiel 2**

Isolation des Natriumsalzes von X-14934A

Stufe A: Zur gesamten Brühe der 60 Gallonen (227,1 Liter) Fermentation, wie sie in Beispiel 1 beschrieben ist, wird nach 139 Stunden Wachstum ein gleiches Volumen Essigester hinzugegeben. Nach Rühren während einer Stunde wird die Lösungsmittelphase abgetrennt, und die wässrige Phase wird erneut mit einem gleichen Volumen Essigester extrahiert. Die zwei Lösungsmittelphasen werden vereinigt und unter vermindertem Druck auf ein Volumen von 2,6 l eingeengt.

Stufe B: Der Essigesterextrakt wird weiterhin zu einem Oel konzentriert. Das Oel wird in n-Hexan gelöst und fünfmal mit einem gleichen Volumen von Acetonitril extrahiert gefolgt von einer Extraktion mit Acetonitril/Methanol (9 : 1). Die Acetonitril und Acetonitril/Methanol (9 : 1) Extrakte werden vereinigt, worauf das Lösungsmittel unter vermindertem Druck entfernt wird. Der so erhaltene Rückstand wird in Essigester gelöst und seinerseits mit 1N Salzsäure, $Na_2CO_3$ (gesättigt bei Raumtemperatur) und Wasser gewaschen. Die Lösungsmittelphase wird über Natriumsulfat getrocknet und unter vermindertem Druck zu einem Oel (38 g) konzentriert.

Stufe C: Das gemäss Stufe B erhaltene Oel wird in Methylenchlorid gelöst und an einer mit Methylenchlorid beschichteten 500 g Silicagelsäule (Davison grade 62) chromatographiert. Die Kolonne wird mit 2 l Methylenchlorid, 4 l Essigester/Hexan (7 : 3), 2 l Essigester/Methanol (95 : 5) und 2 l Essigester/Methanol (9 : 1) eluiert.

Es werden 20 ml Fraktionen gesammelt, und die Fraktionen Nummern 490-512 werden vereinigt. Das Lösungsmittel wird unter vermindertem Druck entfernt, und der Rückstand (8 g) wird erneut chromatographiert an einer Methylenchlorid beschichteten 250 g Silicagelkolonne. Die Kolonne wird mit 2 l Diäthyläther, 2 l Diäthyläther/Aethanol (20 : 0,5) eluiert. Es werden 20 ml Fraktionen gesammelt.

Stufe D: Die Fraktionen Nummern 60-100 der 250 g Siliagelkolonne gemäss Stufe C werden vereinigt, und das Lösungsmittel wird unter vermindertem Druck entfernt. Durch Kristallisation aus Acetonitril/Wasser erhält man kristallines Antibiotikum X-14934A als Natriumsalzdihydrat vom Schmelzpunkt 156-158°C. Mikroanalyse berechnet für $C_{48}H_{79}O_{15}Na.2H_2O$ (955.18): Berechnet: C 60,36, H 8,76, Na 2,41, $H_2O$ 3,77. Gefunden: C 60,62, 60,90; H 8,96, 9,11; Na 2,34, $H_2O$ 4,52.

**Beispiel 3**

Herstellung des Rubidiumsalzes von X-14934A

Eine Lösung von 100 mg Antibiotikum X-14934A Natriumsalz in Methylenchlorid wird erst mit IN HCl dann mit Wasser und dann viermal mit einer wässrigem Lösung von Rubidiumhydroxid gewaschen. Die Lösungsmittelphase wird durch Filtrieren durch Celit getrocknet, unter vermindertem Druck konzentriert und dann aus Acetonitril durch Zugabe von Wasser kristallisiert. Durch Rekristallisation erhält man Kristalle die für Röntgenstrahlanalyse geeignet sind. Analyse berechnet für $(C_{48}H_{79}O_{15})_2Rb(H_2O)_2$: C 60,25, H 8,53, Rb 4,47, $H_2O$ 1,88. Gefunden: C 59,73, H 8,41, Rb 4,63, $H_2O$ 1,74.

Die antimikrobielle Aktivität des Antibiotikums X-14934A wird in der nachfolgenden Tabelle gezeigt:

| | Organismus<br>ATCC No. | Minimale inhibierende Konzentration (MIC)* (MCG/ML) |
|---|---|---|
| G-Stäbchen | Pseudomonas aeruginosa 8705 | >1000 |
| | Proteus vulgaris 6380 | >1000 |
| | Escherichia coli 27856 | >1000 |
| | Klebsiella pneumoniae 27858 | >1000 |
| | Serratia marcescens 27857 | >1000 |
| | Serratia sp. 93 | >1000 |
| | Acinetobacter calcoaccticus 10153 | >1000 |
| G+Kokken | Steptococcus faecium ATCC 8043 | 0,9 |
| | Staphylococcus aureus 6538P | 1,9 |
| | Micrococcus luteus 9341 | 7,9 |
| G+Stäbchen | Bacillus megaterium 8011 | 3,9 |
| | Bacillus sp. E 27359 | 0,45 |
| | Bacillus subtilis 558** | 3,9 |
| | Bacillus sp. TA 27860 | 3,9 |
| G+Filamente | Mycobacterium phlei 355 | 7,9 |
| | Streptomyces cellulosae 3313 | 15,7 |
| Schimmelpilze | Paecilomyces varioti 25820 | 62,5 |
| | Penicillum digitatum 26821 | 125 |
| Hefen | Candida albicans 477** | 7,9 |
| | Saccharomyces cerevisiae 4226 | 125 |

*Mindestkonzentration bei welcher bei der gut bekannten Agar-Diffusionsmethode immer noch eine Inhibitionszone ersichtlich ist.

**NRRL Nummer

Wie oben gezeigt besitzen das Antibiotikum X-14934A und seine Salze die Eigenschaft, das Wachstum gewisser Grampositiver Bakterien negativ zu beeinflussen. Das Antibiotikum X-14934A ist wertvoll in

Waschlösungen für sanitäre Zwecke, wie z. B. in Lösungen zum Händewaschen, Lösungen zur Reinigung von Geräten, Gängen oder von Einrichtungen kontaminierter Räume oder Laboratorien. Es ist ebenfalls wertvoll, das Wachstum sensitiver Organismen in Plattenverfahren und in anderen mikrobiologischen Medien zu unterdrücken.

Das Antibiotikum X-14934A zeigt auch Wirksamkeit gegen Treponema hyodysenteriae, einen die Schweinedysenterie verursachendem Mittel. Es wurde eime erhebliche Aktivität für das Antibiotikum bei Konzentrationen unterhalb einem mcg/ml gefunden.

Antibiotikum X-14934A zeigt auch Aktivität als kokzidiostatisches Mittel.

Diese anti-Kokzidiosewirkung wird an Laborkücken wie folgt gezeigt:

Testmethode

Dieser Test verwendet 10 Kücken pro Wirkstoffgruppe. 10 Kücken werden als Gewichtskontrolle und 10 Kücken als infizierte Kontrolle verwendet. Der Wirkstoff wird 48 Stunden vor der Infektion gegeben. 1 g des Testwirkstoffes wird in einem mechanischem Mischer mit einer genügenden Menge Kückenfutter gemischt um die gewünschte Dosis zu erhalten. Die Infektion besteht aus ungefähr 200,000 Oocyten, welche oral mit einer Pipette gegeben werden. Der Test dauert elf Tage. Die überlebenden Vögel werden autopsiert und auf grobe Lesionen im Caecum untersucht. Die Testvögel werden berechnet nach der Anzahl der überlebenden und der Anzahl der caecalen Lesionen. Die Resultate sind als Durchschnittsausmass der Infektion ausgedrückt (A.D.I.). Ein Durchschnittsausmass von Infektion von weniger als 2,5 wird als signifikant betrachtet.

Aktivität gegen E. Tenella

| Antibiotikum | Konzentration im Futter, ppm | Gewichtszunahme % | Sterblichkeit % | mittleres Ausmass der Infektion |
|---|---|---|---|---|
| Uninfizierte unbehandelte Kontrollgruppe | 0 | 100 | 0 | 0,0 |
| infizierte unbehandelte Kontrollgruppe | 0 | 60 | 20 | 3,0 |
| Lasalocid | 75 | 98 | 0 | 0,0 |
| X-14934A | 100 | 21 | 0 | 0,0 |
| | 25 | 25 | 0 | 0,0 |
| | 10 | 101 | 0 | 0,6 |

Aktivität gegen Mischinfektion*

| | | | | oben | Mitte | Caecum |
|---|---|---|---|---|---|---|
| Uninfizierte unbehandelte Kontrolle | 0 | 100 | 0 | 0,0 | 0,0 | 0,0 |
| Infizierte unbehandelte Kontrolle | 0 | 34 | 20 | 3,0 | 3,0 | 3,1 |
| X-14934A Natriumsalz | 5 | 44 | 40 | 3,1 | 3,1 | 3,2 |
| | 10 | 65 | 20 | 2,7 | 2,4 | 2,7 |
| | 15 | 62 | 0 | 2,0 | 2,0 | 2,0 |

*500,000 Oocyten von gemischten Eimeria Spezies umfassend einen Monensin-resistenten E. Tenella-Stamm.

Die kokzidiostatischen Zusammensetzungen gemäss vorliegender Erfindung enthalten als aktiven Bestandteil kristallines Antibiotikum X-14934A oder ein Salz davon oder die getrocknete unfiltrierte Kulturbrühe und werden durch Mischen des Wirkstoffes mit einem inerten Bestandteil hergestellt. Der inerte Bestandteil kann ein Futtermittel, Streckmaterial oder dergleichen umfassen. Beim Ausdruck "inerter Bestandteil" wird ein Material verstanden, welches nicht als antiparasitisches Mittel wirkt, z. B. ein Kokzidiostatikum, welches bezüglich des Wirkstoffes inaktiv ist und welches gefahrlos den zu behandelnden Tieren eingegeben werden kann und welches somit inaktiv für die Zwecke der vorliegenden Erfindung ist.

Wenn der Wirkstoff oral als eine Komponente des Futters Kokzidiose verdächtigem Hausgeflügel, im speziellen Truthühnern und Kücken, verabreicht wird, kontrolliert er die Krankheit effektiv entweder durch Verhütung der Krankheit oder durch Heilung der Krankheit nach dem sie ausgebrochen ist. Im weiteren behält das behandelte Hausgeflügel entweder das Gewicht oder es gewinnt sogar noch an Gewicht, wenn man es mit Kontrollen vergleicht. So also kontrollieren die erfindungsgemässen Zusammensetzungen nicht nur Kokzidiose, sondern bewirken eine Verbesserung der Effizienz der Verwertung von Futter zur Gewichtszunahme.

Die aktuelle Konzentration des Wirkstoffes im tierischen Futter kann nach einzelnen Wünschen eingestellt werden und kann über einen weiten Bereich variieren. Der limitierende Faktor der Konzentration ist, dass eine Konzentration verabreicht wird, bei der eine genügende Menge an Wirkstoff verabreicht wird, um die gewünschte Kontrolle der Kokzidiose zu erreichen, und die maximale Konzentration ist die, bei der die Menge an Zusammensetzung keine ungünstigen oder unerwünschten Nebeneffekte bewirkt.

So zum Beispiel enthält eine Futtervormischung oder ein vollständiges Futter genügend Wirkstoff, um eine Aufnahme von ungefähr 1 ppm bis ungefähr 20 ppm (Gewicht) mit der täglichen Futteraufnahme zu erreichen. Vorzugsweise werden 10 ppm bis 15 ppm verwendet. Im allgemeinen ist ungefähr 1 bis ungefähr 15 ppm Wirkstoff genügend für den Zweck die Kokzidiose zu kontrollieren und zu bekämpfen. Mengen grösser als 20 ppm, obwohl sie gegen Kokzidiose wirksam sind, zeigen in der Regel keine verbesserten Resultate gegenüber den bevorzugten ppm-Bereichen und beeinträchtigen in einigen Fällen das Wachstum, die Futteraufnahme und die Mortalität negativ.

Die optimale Dosis wird natürlich je nach Grösse des Tieres variieren. Wenn das Antibiotikum X-14934A gemäss vorliegender Erfindung zur Behandlung oder Verhütung von Kokzidiose verwendet wird, kann es zuerst mit einem Futterbestandteil oder einem Futterträger zu einer Futterzusatzvormischung, einem Futterkonzentrat oder einem Futterzusatzsupplement verarbeitet oder gemischt werden. Ein Futterzusatz, Konzentrat oder Vormischung ist ein Bestandteil welcher geeignet ist, um zu einem vollständigen Futter verdünnt zu werden, d.h. also ein Bestandteil, welcher zur Verabreichung einer einzigen Ration geeignet ist. Eine Futterzusatzergänzung ist ein Gegenstand, der für die Direktkonsumation durch das Tier geeignet ist, oder der zu einem vollständigen Futter weiter verdünnt werden kann oder der als eine Ergänzung zu anderen Rationen eingenommen und werwendet werden kann. Futterzusatzergänzungen, Konzentrate und Vormischungen enthalten relativ grosse Prozentsätze von Kokzidiostatikum, d.h. also von Wirkstoff, und werden im herkömmlicher Weise hergestellt durch Hinzufügen des Wirkstoffes zu einem geeigneten Träger und durch Mischen in einer Weise, die eine einheitliche Verteilung des Kokzidiostatikums im Träger liefert. Geeignete Träger sind solche, welche bezüglich des Wirkstoffs inert sind und welche von den zu behandelnden Tier sicher eingenommen werden können. Typische dieser Träger sind käuflich erhältliches Geflügelfutter, Grundgetreidekörner, Körnerbeiprodukte, Pflanzenproteinkonzentrate (Soya, Erdnüsse etc.) Fermentationsbeiprodukte, Salz, Kalkstein, anorganische Verbindungen und dergleichen sowie Mischungen davon. Flüssige Dispersionen können erhalten werden unter Verwendung von Wasser, pflanzlichen Oelen, vorzugsweise unter Verwendung eines oberflächenaktiven Mittels, eines Emulgiermittels oder dergleichen wie Äthylendiamintetraessigsäure etc. und Lösungsvermittlern. Jeder geeigneter Träger oder Füllstoff kann als inerter Bestandteil in der festen Form des Antiparasitenmittels dienen, sofern er bezüglich des Wirkstoffs inert ist und bezüglich des Tieres, welchem er verabreicht wird, nicht toxisch ist.

Der Wirkstoff kann zu einem Brei, zu einer Pille oder zu jeder gewünschten Form mit dem inerten Träger oder Füllstoff nach herkömmlichen Techniken gemischt werden. Beispielsweise können Zusammensetzungen durch feines Verreiben oder Pulverisieren des Wirkstoffes und der inerten Bestandteile unter Verwendung kommerziell erhältlicher Zerkleinerer oder Pulverisierer in Ab- oder Anwesenheit des Futtermaterials geformt werden. Sofern das Futtermaterial beim Zerkleinern oder Pulverisieren nicht anwesend ist, kann das erhaltene Material gemäss vorliegender Erfindung in jedes herkömmlich erhältliche Futtermittel verteilt werden. Typisches Geflügelfutter, welches mit dem Wirkstoff gemäss vorliegender Erfindung vermischt werden kann, kann verschiedene Bestandteile enthalten, z. B. Hochenergiekornprodukte wie Getreide, Weizen, Weizenmehl "red dog", Milo, Hafermehl oder dergleichen: Mittel und Niedrigenergiekornprodukte, wie Hafermehl, Gerste, Weizenmehl, Mittelmehl, Standardmittelmehl oder dergleichen: stabilisierte Fette, pflanzliche Proteine, wie Soyabohnenmehl, Weizenklebermehl, Erdnussmehl oder dergleichen; tierische Proteine wie Fischmehl, lösliche Fischbestandteile, Fleischbrocken oder dergleichen; UPF-(undefinierter Wachstumsfaktor) Quellen und andere B-Vitaminträger, wie Trockenmilchprodukte, getrocknete Brauhefe, getrocknete Schlempe, Fermentationsflüssigkeiten oder dergleichen; dehydriertes AlfaAlfa-Mehl, und verschiedene Spezialzusätze wie zusätzliches Riboflavin, Vitamin $B_{12}$, Calciumpantothenat, Niacin, Cholin, Vitamin K und Vitamin E oder dergleichen, sowie stabilisiertes Vitamin A, Vitamin $D_3$ (D-aktivierte tierische Steroide); Calcium und Phosphorergänzungen, wie Dicalciumphosphat, mit Dampf behandeltes Knochenmehl, entfluorinisiertes Phosphat, Kalkstein oder dergleichen; jodiertes Salz, Mangansulfat, Zinkcarbonat, ein antibiotisches Futterergänzungsmittel; Methionin oder dessen Hydroxyanaloga und ein Antioxidant.

Wie aus obigem ersichtlich wird, sind die kokzidiostatischen Zusammensetzungen für die orale Einnahme beabsichtigt. Sie können dem normalen Futterbedarf des behandelten Tieres beigesetzt werden oder können auf andere Weise verabreicht werden, wie z. B. durch zwangsweise Verabreichen derselben in Form einer Tablette, Pille oder eines Bolus. Die Verabreichung des Wirkstoffes muss unter Berücksichtigung des spezifischen Tieres sowie unter Berücksichtigung der Tierzuchtpraktiken erfolgen.

Ein geeignetes, Wirkstoff enthaltendes Geflügelfutter, welches als Anfangsfutter für Brüter geeignet ist, wird durch Mischen von 10-15 ppm (Gewicht) des Antibiotikums X-14934A in eine Basis Geflügelration die die nachfolgende Zusammensetzung hat, erhalten.

| Bestandteile | | |
|---|---|---|
| Weizenmehl, Nr. 2, gelb | Pfund/Tonne | 1,123 |
| Stabilisiertes Fett oder pflanzliches Oel | " | 60 |
| Soyabohnenölmehl (niedriger Fasergehalt, 50 % Protein) | " | 480 |
| Weizenklebermehl | " | 50 |
| Fischmehl, mit Antioxidantien behandelt, 60 % Protein | " | 30 |
| lösliches Fischmaterial, Trockenbasis | " | 10 |
| Fleisch- und Knochenstücke, 50 % Protein | " | 140 |
| getrocknete Rückstände der Maisdestillation | " | 50 |
| Alfaalfamehl, 17 % Protein 10,000 A/lb | " | 30 |
| jodiertes Salz | " | 5 |
| Mangansulfat, Futtergrad | " | 0,75 |
| Zinkcarbonat oder Oxid | " | 0,25 |
| Riboflavin | gr. | 3 |
| Vitamin $B_{12}$ | mg | 6 |
| Calciumpantothenat | gr. | 5 |
| Niacin | " | 30 |
| stabilisiertes Vitamin A (USPI units) | " | 6,000,000 |
| Vitamin $D_3$ I.C. | " | 650,000 |
| Vitamin E Acetat I.U. | " | 5,000 |
| Vitamin E (Menadionnatrium-bisulfit) | " | 2 |
| DL-Methionin oder analoges | Pfund/Tonne | 1 |
| Antioxidants (Aethoxyquin oder butyliertes Hydroxytoluol | " | 0,25 |

Aehnliche Futtermittel können hergestellt werden mit anderen Konzentrationen des Antibiotikums, und zwar sowohl unter Verwendung des reinen Antibiotikums wie von getrockneter unfiltrierter Kulturbrühe, und zwar in einer Menge, die dieselbe Konzentration an aktivem Antibiotikum ergibt.

Antibiotikum X-14934A zeigt auch eine Erhöhung der Futterverwertung bei Wiederkäuern. Die Verbindung ist aktiv, und zwar verschiebt sie die flüchtigen Fettsäuremengen zu erhöhten Propionatspiegeln, und zwar bei Dosen von ungefähr 50 ppm.

**Patentansprüche** für die Vertragsstaaten BE CH DE FR GB IT LI LU NL SE

1. Eine Verbindung der Formel

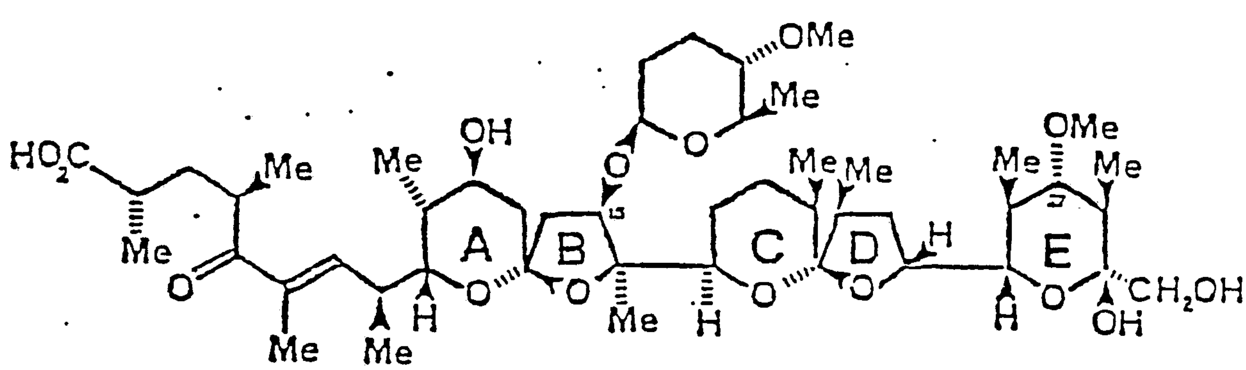

I

und Salze davon.

2. Verbindungen der Formel I, worin das Salz das Natriumsalz ist.

3. Verfahren zur Herstellung einer Verbindung der Formel I gemäss Patentanspruch 1 oder eines Salzes davon dadurch gekennzeichnet, dass man einen Stamm von Streptomyces X-14934 in einer wässrigen

Kohlehydratlösung enthaltend ein Stickstoffnährmittel unter submergen aeroben Bedingungen kultiviert und darauf die Verbindung der Formel I von dieser Lösung isoliert und erwünschtenfalls ein Salz der Verbindung der Formel I bildet.

4. Ein Kokzidiostatikum oder ein Mittel zur Erhöhung der Futterverwertung enthaltend eine wirksame Menge einer Verbindung der Formel I gemäss Anspruch 1 oder eines Salzes davon.

**Patentansprüche** für den Vertragsstaat Oesterreich

1. Verfahren zur Herstellung einer Verbindung der Formel

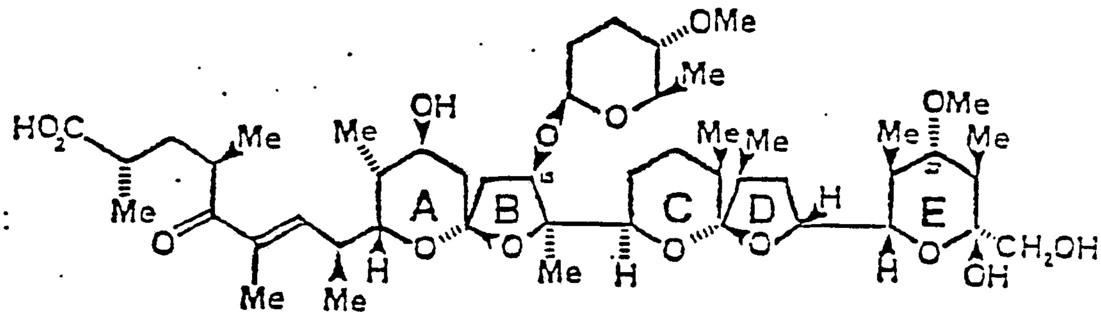

I

dadurch gekennzeichnet, dess man einen Stamm von Streptomyces X-14934 in einer wässrigen Kohlehydratlösung enthaltend ein Stickstoffnährmittel unter submergen aeroben Bedingungen kultiviert und hernach die Verbindung der Formel I aus dieser Lösung isoliert und falls erwünscht, ein Salz der Verbindung der Formel I bildet.

2. Verfahren nach Anspruch 1, dadurch gekennzeichnet, dass man das Natriumsalz der Verbindung der Formel I herstellt.

**Claims** for the contracting states BE, CH, DE, FR, GB, IT, LI, LU, NL, SE

1. A compound of the formula

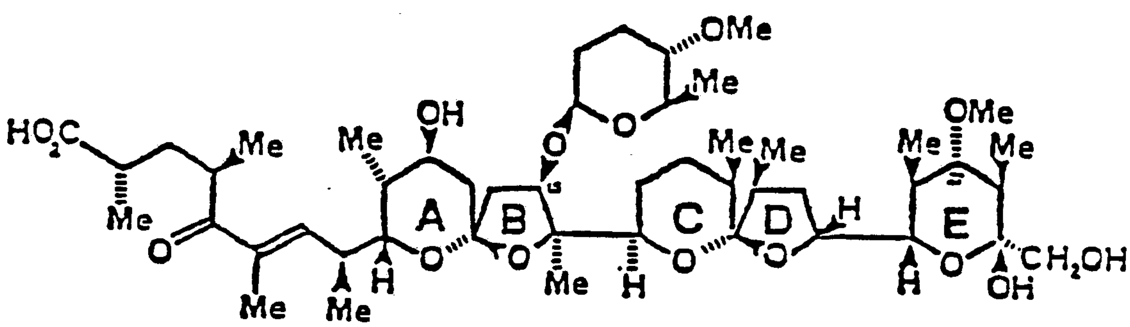

I

and salts thereof.

2. Compounds of formula I, wherein the salt is the sodium salt.

3. A process for the manufacture of a compound of formula I in accordance with claim 1 or of a salt thereof, characterized by cultivating a strain of Streptomyces X-14934 in an aqueous carbohydrate solution containing a nitrogenous nutrient under submerged aerobic conditions and thereupon isolating the compound of formula I from this solution and, if desired, forming a salt of the compound of formula I.

4. A coccidiostat or a composition for increasing feed utilization containing an effective amount of a compound of formula I in accordance with claim 1 or of a salt thereof.

**Claims** for the Contracting State AT

1. A process for the manufacture of a compound of the formula

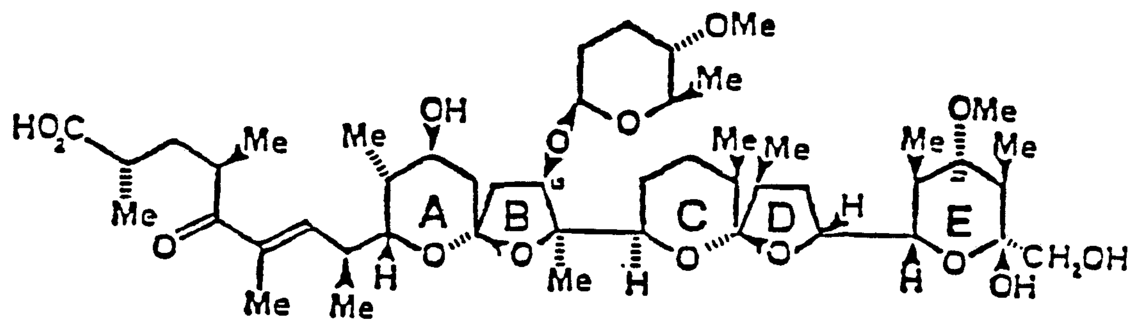

I

characterized by cultivating a strain of Streptomyces X-14934 in an aqueous carbohydrate containing a nitrogenous nutrient under submerged aerobic conditions and thereafter isolating the compound of formula I from this solution and, if desired, forming a salt of the compound of formula I.

2. A process according to claim 1, characterized in that the salt of the compound of formula I is manufactured.

**Revendications** pour les Etats Contractants BE CH DE FR GB IT LI LU NL SE

1. Un composé de formule

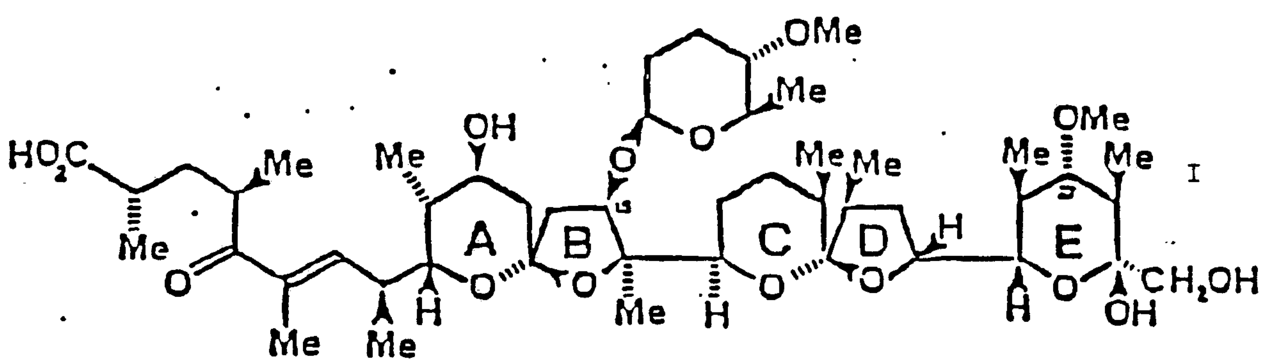

I

et ses sels.

2. Composés de formule I, à l'état de sels de sodium.

3. Procédé pour la préparation d'un composé de formule I selon la revendication 1 ou d'un sel d'un tel composé, caractérisé en ce que l'on soumet une souche de Streptomyces X-14934 à une culture aerobie submergée dans une solution aqueuse d'hydrate de carbone contenant une substance nutritive azotée puis on isole le composé de formule I de cette solution et, si on le désire, on forme un sel du composé de formule I.

4. Un coccidiostatique ou un produit pour améliorer la valorisation des aliments, contenant une quantité efficace d'un composé de formule I selon la revendication 1 ou d'un sel d'un tel composé.

**Revendications** pour l'Etat contractant AT

1. Procédé de préparation d'un composé de formule

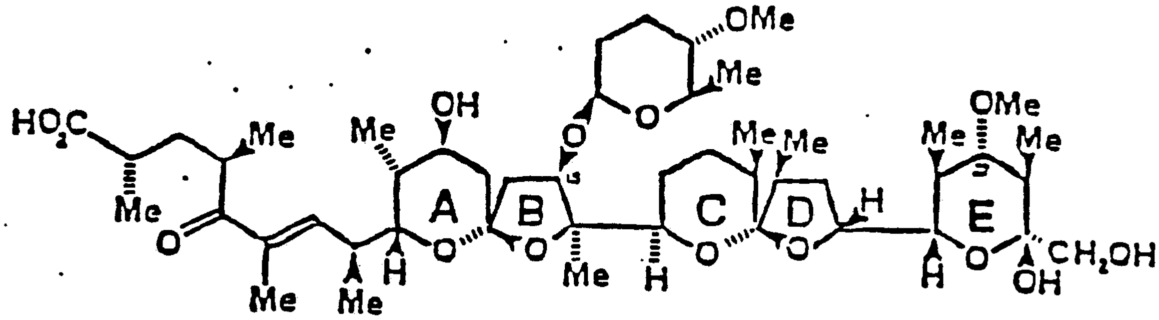

I

caractérisé en ce que l'on soumet une souche de Streptomyces X-14934 à culture aérobie submergée dans une solution aqueuse d'hydrate de carbone contenant une substance nutritive azotée, on isole ensuite le composé de formule I de cette solution et, si on le désire, on forme un sel du composé de formule I.

2. Procédé selon la revendication 1, caractérisé en ce que l'on prépare le sel de sodium du composé de formule I.